# EUROPÄISCHE PATENTSCHRIFT

(11) **EP 1 894 602 B1**
(45) Veröffentlichungstag und Bekanntmachung des Hinweises auf die Patenterteilung: **10.11.2010**
(21) Anmeldenummer: 07112501.7
(22) Anmeldetag: 16.07.2007
(51) Int. Cl.: A61Q 1/10, A61K 8/37, A61K 8/92

(54) **Mascara mit einem Gehalt an synthetischen Wachs, Bienenwachs und synthetischen Esterwachs**
Mascara containing synthetic wax, bee wax and synthetic ester wax
Mascara comprenant une cire synthétique, une cire d'abeille et un cire synthétique d'ester

(30) Priorität: 16.08.2006 DE 102006039110
(43) Veröffentlichungstag der Anmeldung: 05.03.2008
(73) Patentinhaber: Beiersdorf Aktiengesellschaft, 20253 Hamburg (DE)
(72) Erfinder: Jaberi Motlagh, Sepideh, 22459, Hamburg (DE); Schwanke, Frank, 20149 Hamburg (DE); Reuter, Bettina, 25451, Quickborn (DE)

(56) Entgegenhaltungen:
- EP-A- 0 795 312
- EP-A- 1 396 259
- EP-A- 1 466 579
- EP-A- 1 563 824
- EP-A- 1 698 337
- EP-A- 1 847 254
- WO-A-02/30368
- WO-A-02/47622
- WO-A-2004/009043
- FR-A- 2 833 163
- FR-A- 2 844 195

## Beschreibung

Die vorliegende Erfindung betrifft kosmetische Zubereitung mit einem Gehalt an Synthetikwachs, Bienenwachs und synthetischen Esterwachsen zum Färben und Formen von keratinischem Material.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares und der Wimpern und Augenbrauen dürfte allerdings heute darin bestehen, das Aussehen des Menschen in charakteristischer Weise mitzugestalten. Ähnlich wie die Haut erfüllen sie eine soziale Funktion, da sie über ihr Erscheinungsbild erheblich zu zwischenmenschlichen Beziehungen und zum Selbstwertgefühl des Individuums beitragen.

Die Haare, d.h. auch die Augenbrauen und Wimpern, bestehen aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die ein ganzes Haar umhüllt.

Das Färben des menschlichen Haupthaares und insbesondere der Augenbrauen und Wimpern ist ein wichtiges Gebiet der dekorativen Kosmetik. Eine dauerhafte Färbung, wie es auch beim Haupthaar oftmals erwünscht ist, sollte auch durch intensives Waschen nicht wieder aus dem Haar entfernbar sein und in Hinsicht auf Umwelteinflüsse wie UV-Licht, Schweiß und Feuchtigkeit eine hohe Echtheit aufweisen. Entsprechende Färbemittel werden in der Regel auf Basis von Oxidationsfarbstoffvorprodukten formuliert.

Im Rahmen moderner Modetrends besteht aber immer häufiger das Bedürfnis nach Farbeffekten am Haar, die auch ohne Inanspruchnahme eines Friseurs oder Kosmetikers auf einfache Weise erzielbar und ebenso leicht, z. B. durch einmaliges, übliches Waschen des Haares, wieder zu entfernen sind. Insbesondere sollen sich die entsprechenden Farbeffekte auch auf einzelne Haarsträhnen oder Haarpartien beschränken lassen. Weiterhin ist es in hohem Maße wünschenswert, wenn sich die auch noch auf der fertig gestylten Frisur anbringen lassen.

Eine Produktgruppe, die prinzipiell diese Anforderungen erfüllen könnte, stellen die Wimperntuschen dar, die auch unter dem Namen "Mascara" (wohl letztlich von arab.: 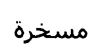 (mashara) = Possenreißer, Harlekin, auch: Harlekinade; vgl.: Maskerade), bekannt sind. Augen Make-up, insbesondere Mascaraprodukte (Wimperntusche), haben im dekorativen Marktsegment eine große Bedeutung. Mit Mascara, aber auch mit Augenbrauenstiften, kann man die Wimpern und Brauen optisch effektvoll betonen und den Augen dadurch mehr Ausdruck verleihen.

Die Qualität einer Mascara lässt sich messen an einem guten und leichten Auftrag, einer angemessenen Trockenzeit und einer guten Haftfestigkeit. Weiterhin sollte eine Mascara sich nicht nachteilig auf das Wimpernhaar auswirken, z.B. nicht austrocknend wirken oder die Brüchigkeit erhöhen. Im Gegenteil: Es ist wünschenswert, eine Mascara zu erhalten, die neben den genannten Aspekten sogar noch pflegende Eigenschaften aufweist und den Wimpern Schwung und Elastizität verleiht.

Ein großes Problem stellen dabei jedoch die Haftfestigkeit der Mascara auf dem Haar /den Wimpern dar. Die Konsistenz, die Trockenzeit und der auf der Wimper verbleibende Film einer Mascara muss so beschaffen sein, dass für ein gute optische Betonung ausreichend Masse auf jeder Wimper haften bleibt, mehrere Wimpern jedoch nicht zu so genannten "Fliegenbeinen" zusammenkleben. Für die optische Verschönerung ist die Volumen gebende bzw. verlängernde Wirkung einer Mascara besonders wichtig.

Insbesondere sollte auch nach mehreren Stunden Tragezeit der getrocknete Film nicht "bröckelig" werden und sich nicht durch Feuchtigkeit, Sebum oder z.B. andere öllöslichen Bestandteile einer Pflegecreme anlösen lassen, so dass sich keine dunklen Ränder um die Augen bilden. Für diese negativen Eigenschaften sind meist die in den Mascaraformulierungen enthaltenen Füllstoffe, wie z.B. Talkum, Silikate, oder Fasern, wie z.B. Nylon, Rayon, verantwortlich.
Durch die Einstellung der Trockenzeit wird die Zeit definiert, die der Anwenderin zum (auch mehrmaligen) Auftragen bleibt, bevor der Mascarafilm getrocknet / fixiert ist. Die Trockenzeit liegt bei guten Mascarazubereitungen und unter normalen Bedingungen bei ca. 1-1,5 Minuten.

Dabei ist der Einsatz eines Filmbildners, unter diesen Oberbegriff fallen auch einige als Wachs bezeichnete Polymere, zur Steuerung der vorgenannten Eigenschaften von besonderer Bedeutung.

Die Verwendung von Filmbildnern in haarkosmetischen Zubereitungen, insbesondere Mascarazubereitungen, ist an sich bekannt. Die DE-OS 197 46 468 beschreibt eine Mascara, enthaltend mindestens ein Weiß- oder Farb-Pigment sowie übliche kosmetische Bestandteile in einer wässrigen Grundlage, dadurch gekennzeichnet, dass sie ein Wachs mit einem Schmelzpunkt von mindestens 60 °C und ein nichtionogenes synthetisches Polymer enthält. Die am angegebenen Ort als bevorzugt dargestellten Polymere werden beispielsweise gewählt aus der Gruppe der nichtionogenen, synthetischen Polymere, insbesondere Polyvinylalkohole, Poly(N-vinyl-formamid), Poly(N-vinyl-caprolactam), Polyvinylpyrrolidone sowie Copolymere des Vinylpyrrolidons mit mindestens einem weiteren nichtionogen Monomer. Als bevorzugt werden Vinylacetat Polyvinylalkohole sowie Polyvinylpyrrolidone, Copolymere und Mischungen dieser Polymeren dargestellt.

Viele auf dem Markt befindlichen Produkte besitzen hoch schmelzende Wachse, welche zur Volumengebung und Verdickung beitragen (sog. Volumen-Mascara). Diese Produkte trocknen jedoch sehr schnell und werden dadurch bröckelig.

Weiterhin ist bei der Verwendung von Mascara erwünscht, dass den Wimpern ein Schwung verliehen wird und nach der Antrocknung des Mascaras die Wimpern elastisch bleiben.

Im Stand der Technik sind bereits eine Reihe von Mascarazubereitungen bekannt, die Sythetikwachs, Bienenwachs, synthetisches Esterwachs und Filmbildner enthalten ( WO 2002047622 A2, EP 1396259 A2, WO 2004009043 A1, FR 2844195 A1, EP 0795312 A1, EP 1563824 A1, FR 2833163 A1 und WO 2002030368 A2), wobei als Synthetikwachs Polyolefinwachse bzw. Mischungen aus Polyolefinwachsen und Fischer-Tropsch-Wachsen zum Einsatz kommen.

Aus der EP 1466579 A2 sind Mascarazubereitungen bekannt, die die Sythetikwachs, Bienenwachs, synthetisches Esterwachs, Filmbildner und Emulgator enthalten.

Aufgabe der vorliegenden Erfindung war, die Nachteile des Standes der Technik zu beseitigen. Insbesondere sollten verbesserte kosmetische Zubereitungen zur Verfügung gestellt werden, die sich durch bessere Filmbildung auszeichnen, indem der getrocknete Film elastische und formgebende Eigenschaften aufweist und nicht bröckelig wird. Insbesondere ist jedoch die Verbesserung, sprich optische Verschönerung, in Bezug auf Volumengebung Aufgabe der vorliegenden Erfindung.

Weiterhin sollten Zubereitungen zur Verfügung gestellt werden, die sich nicht nachteilig auf das Wimpernhaar auswirken, z.B. die Wimpernhärchen nicht verkleben und so genannte "Fliegenbeine" ausgebildet werden.

Erfindungsgemäß werden diese Aufgaben gelöst und die Nachteile des Standes der Technik durch Formulierungen gemäß Hauptanspruch beseitigt.

Gegenstand der Erfindung sind also Mascarazubereitungen, **dadurch gekennzeichnet, dass** sie mindestens einen Filmbildner, mindestens einen Emulgator und eine Kombination aus mindestens einem synthetischem Wachs (Synthetikwachs), Bienenwachs und mindestens einem synthetischen Esterwachs enthalten, wobei das Synthetikwachs ein Fischer-Tropsch Wachs mit einer Kettenlänge von 23-64 Kohlenwasserstoffatomen, einer Molmasse von > 300 g/mol und einer Viskosität von > 5 mn²/s bei 99°C ist. Durch die Kombination des hoch schmelzenden Synthetikwachses und des nierdrig schmelzenden Esterwachses kann man eine maximale Volumengebung erzielen.

Alleiniger Einsatz dieser Kombination aus Volumen gebenden Wachsen führt jedoch zu hohen Viskositäten woraus folgt, das die Mascaramasse sehr fest und hart ist, was sich negativ auf die Applizierbarkeit auswirkt und zum schnelleren Austrocknen führt. Diesen hohen Viskositäten kann durch Zumischung von Bienenwachs, einem plastischen Wachs, entgegen gewirkt werden.

Durch die Kombination von Synthetikwachs, Bienenwachs in Verbindung mit synthetischem Esterwachs werden die Nachteile des Standes der Technik deutlich vermindert und eine geschmeidige Zubereitung bereitgestellt. Diese zeichnet sich insbesondere dadurch aus, das die Wimpern nicht austrocknen und das die Zubereitung trotz höherer Viskosität beim Applizieren geschmeidig bleibt.

"Wachs" ist - ähnlich wie "Harz" - eine Sammelbezeichnung für eine Reihe natürlicher oder künstlich gewonnener Stoffe, die in der Regel folgende Eigenschaften aufweisen: bei 20 °C knetbar, fest bis brüchig hart, grob- bis feinkristallin, durchscheinend bis opak, jedoch nicht glasartig, über 40 °C ohne Zersetzung schmelzend, schon wenig oberhalb des Schmelzpunkts verhältnismäßig niedrigviskos und nicht fadenziehend, stark temperaturabhängige Konsistenz und Löslichkeit und unter leichtem Druck polierbar. Ist in Grenzfällen bei einem Stoff mehr als eine der vorstehend genannten Eigenschaften nicht erfüllt, so ist er kein Wachs im Sinne dieser Definition. Wachse unterscheiden sich von ähnlichen synthetischen oder natürlichen Produkten (z. B. Harzen, plastischen Massen usw.) hauptsächlich darin, dass sie in der Regel etwa zwischen 50 und 90 °C, in Ausnahmefällen auch bis zu etwa 200 °C, in den schmelzflüssigen, niedrigviskosen Zustand übergehen und praktisch frei von Asche bildenden Verbindungen sind.

Nach Festlegung der Deutschen Gesellschaft für Fettwirtschaft (Fette, Seifen, Anstrichmittel, 76, 135 [1974]) werden zur Kennzeichnung des Begriffes "Wachs" in der Regel die mechanisch-physikalischen Eigenschaften der Wachse, welche für ihre Verwendung maßgebend sind, herangezogen, während für die Begriffsbestimmung die jeweilige chemische Zusammensetzung unberücksichtigt bleibt.

Erfindungsgemäß werden Polyethylenwachse, eingesetzt, welche nach dem Fischer-Tropsch-Verfahren hergestellt werden eingesetzt. Diese synthetischen Wachse weisen im Allgemeinen eine Kettenlänge von 23 bis 64 Kohlenstoffatomen und eine Molmasse von > 300 g/mol auf. Die Viskosität der bevorzugten Synthetikwachse beträgt >5 mm²/s bei 99°C.
Die Synthetikwachse sind in den erfindungsgemäßen Zubereitungen bevorzugt in Mengen von 0,5 bis 12,0 Gew.-%, bezogen auf das Gesamtgewicht der Zubereitungen, enthalten. Mengen von 1,0 bis 10,0 Gew.-%, insbesondere von 4,0 bis 8,0 Gew.-%, sind besonders bevorzugt.
Erfindungsgemäß ganz besonders bevorzugt ist Sasolwax C80 einem synthetischen Wachs der Firma Sasol mit sehr linearer Struktur, welches insbesondere den Glanz und die Wischfestigkeit der Mascars zu erhöhen vermag. Im Sasolwax C 80 liegt die maximale Kettenlänge bei 36-39 (Modalwert) Kohlenstoffatomenen, was zu einer Kombination aus hohem Schmelzpunkt, niedriger Viskosität und hoher Härte, auch bei höheren Umgebungstemperaturen, in der Mascarazubereitung führt.

Esterwachs ist die Sammelbezeichnung für Umsetzungsprodukte langkettiger monovalenter Wachssäuren (C22-C34) mit einwertigen Fett- od. Wachsalkoholen (C24-C32).
Vertreter natürlich vorkommender Esterwachse sind z.B. Carnauba-, Candelilla-, Ouricuri-, Zuckerrohr-, Bienen- u. Montanwachs.
Synthetische Esterwachse lassen sich durch Raffination und chemische Abwandlung aus Montanwachs herstellen. Montanesterwachse selbst gehören ebenfalls in diese Klasse und gelten als wertvolle Rohstoffe für chemisch-technische Erzeugnisse.

Im allgemeinen weisen die synthetischen Esterwachse einen Schmelzpunkt unter 85 °C und eine Viskosität von 5 bis 20 mm²/s bei 99°C auf (alle angeführten Viskositäten gemessen nach ISO 3104).

Bevorzugte synthetische Esterwachse Cetylpalmitat, Behenyl Behenate, Glycerinester, Sunflower Wax oder Hydriertes Jojobaöl.

Besonders bevorzugt ist es, als synthetisches Esterwachs Kesterwachs K62 der Firma Koster Keunen einzusetzen. Dieses Wachs ist ein Veresterungsprodukt von langkettig gesättigten Fettalkoholen und Säuren pflanzlicher Herkunft und enthält Ester mit Carbonkettenlängen zwischen 32 und 46 Kohlenstoffatomen, insbesondere zwischen 36 und 44 Kohlenstoffatomen und einer Molmasse von 500 bis 650 g/mol.

Die synthetischen Esterwachse sind in den erfindungsgemäßen Zubereitungen bevorzugt in Mengen von 0,5 bis 12,0 Gew.-%, bezogen auf das Gesamtgewicht der Zubereitungen, enthalten. Mengen von 2,0 bis 10,0 Gew.-%, insbesondere von 4,0 bis 8,0 Gew.-%, sind besonders bevorzugt.

Bienenwachs ist das älteste vom Menschen genutzte Naturwachs und auch heute noch das bedeutendste Wachs tierischen Ursprungs. Bienenwachs (E 901, INCI-Bez.: Beeswax) ist ein Ausscheidungsprodukt aus den Drüsen der Honig-Biene (Apis mellifica), aus dem die Honigwaben aufgebaut werden. Bienenwachs besteht aus einem Gemisch von komplexen Wachsestern (ca. 70%), normalen Fettsäuren u. Hydroxyfettsäuren (13-14%) sowie Kohlenwasserstoffen (10-14%). Die Wachsester enthalten als Alkohol-Komponente zum Beispiel 1-Triacontanol, das insbesesondere mit Palmitin- und Cerotinsäure verestert ist. Als Wachsester von Hydroxyfettsäuren ist zum Beispiel Ceryl-hydroxypalmitat (8-9%) im Bienenwachs vorhanden. 14-Hydroxypalmitinsowie 16-Hydroxy- und 17-Hydroxystearinsäure sind ebenfalls Bestandteile von Bienenwachs. Als freie Wachssäuren kommen zum Beispiel auch Lignocerin-, Cerotinu. Triacontansäure vor.
Bienenwachs ist eine knetbare, feste Masse mit angenehm honigartigem Geruch, einem Schmp. von 61-68°C u. einer Dichte von 0,95-0,96.

Erfindungsgemäß ganz besonders bevorzugt sind Bienenwachse mit einem Schmelzpunkt von 60 bis 68 °C, einer Viskosität ca. 11 mm²/s bei 100 °C, wobei es ca. 70% langkettige Esteralkohole (C24 bis C44) mit Säuren (C16 bis C18) und 13-18% Hydrocarbone insbesondere C25 bis C35, 10-15% freie Wachssäuren C24 bis C32, um 1% freie Alkohole C34 bis C36 und um 0,5-1% Myristolacton enthält. Beispiel für ein Bienenwachs dieser Qualität ist das Bienenwachs weiss' der Fa. Kahl (Typ Art. 2956) welche sich zusätzlich durch seine besondere Reinheit und weiße Farbe auszeichnet. Der sich aus dem Einsatz der zuvor genannten Bienenwachse ergebende Vorteil liegt in der Verbesserung der Ölbindung, Feuchtigkeitsgebung, cremigen Textur und Reduzierung des Oberflächenglanzes.

Das Bienenwachs ist in den erfindungsgemäßen Zubereitungen bevorzugt in Mengen von 0,5 bis 7,0 Gew.-%, bezogen auf das Gesamtgewicht der Zubereitungen, enthalten. Mengen von 1,5 bis 6,0 Gew.-%, insbesondere von 2,5 bis 5,0 Gew.%, sind besonders bevorzugt.

Erfindungsgemäß beträgt der Gehalt an Wachsen 1,5 - 31 Gew.-% (Summe aller eingesetzten Wachse), bezogen auf das Gesamtgewicht der Zubereitungen , bevorzugt 4,5 - 26 Gew.-%, insbesondere von 10,5 - 21 Gew.-%, beträgt.

Die erfindungsgemäßen Zubereitungen enthalten neben Synthetikwachsen, Bienenwachs und synthetischem Esterwachs ferner übliche Emulgatoren und Filmbildner, sowie optional natürliche Wachse, Farb-, Wirk-, Inhalts-, Zusatz- und/oder Hilfsstoffe

Eine wesentliche Komponente der Erfindungsmäßigen kosmetischen Zubereitung ist ein Filmbildnersystem. Das Filmbildnersystem wird von einem Polymer oder mehreren Polymeren, das/die in einem Medium in Dispersion oder gelöst vorliegt, gebildet. Das Polymer kann ein synthetischen oder natürlichen Ursprung haben. Filmbildnersysteme , die besonders geeignet sind, enthalten anionische, kationische, nichtionische und/oder amphotere Polymere aus der Klasse der VP/Hexadecene oder Eicosene Copolymer, Vinylacetat Polyvinylalkohole, Polyvinylalkohole, Poly(N-vinyl-formamid), Poly(N-vinyl-caprolactam), Polyvinylpyrrolidone sowie Copolymere des Vinylpyrrolidons mit mindestens einem weiteren nichtionogen Monomer. Weitere Polymere sind: Polyamide, Polyurethane, Polyether, Siliconpolymere, Acrylpolymere, AcrylateslOctylacrylamide Copolymer, Cellulosederivate, Polysaccharidderivate. Besonders vorteilhaft ist die Verwendung von Acrylatcopolymeren, insbesondere Acrylat/Octylacrylamid Copolymer.

Mascarazubereitungen wie auch die erfindungsgemäße Zubereitung liegen als Emulsionen vor und enthalten einen oder mehrere Emulgatoren. Im Falle der erfindungsgemäßen Zubereitung ist das Emulsionssystem ein Öl-in-Wasser-System (O/W-Emulsion) mit vorzugsweise einem O/W-Emulgator.
O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)n-R',
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ,-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(1 4)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),
Polyethylenglycol(13)cetylether (Ceteth-13), Polyethylenglycol(1 4)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(1 4)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(1 4)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat
Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Ganz bevorzugt werden Fettalkohole wie z.B. Cetyl Alkohol als Emulgatoren verwendet.

Die erfindungsgemäßen Zubereitungen können neben Wachsen, Filmbildnern und Wasser weitere kosmetische Hauptkomponenten und Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, weichmachende, anfeuchtende und/oder feucht haltende Substanzen, rückfettende Agentien, Fette, Öle,, Wachse (die keine synthetischen bzw. FT-Wachse sind), Alkohole, Polyole mit mehr als 4 Kohlenstoffatomen und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, antimikrobielle Stoffe, UV-Absorber, Komplexierungs- und Sequestrierungsagentien, Perlglanzagentien, Polymere, Weichmacher, W/O-Emulgatoren, Elektrolyte, organische Lösungsmittel, Wasser" Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hauptkomponenten und Hilfsstoffe beträgt beispielsweise 88 bis 98,75 Gew.-%, vorzugsweise 90 bis 95 Gew.-%, jeweils bezogen auf das Gesamtgewicht der Zubereitung.

Die erfindungsgemäßen Zubereitungen können zur Bildung der wässrigen Phase der Emulsion Wasser, z.B. 1 bis 80 Gew.-% und/oder Alkohole, z.B. 0,5 bis 10 Gew.-% enthalten, jeweils bezogen auf das Gesamtgewicht der Zubereitungen.

Bevorzugte Emulsions-Mascara im Sinne der vorliegenden Erfindung enthalten als Hauptbestandteile synthetische und natürliche Wachse, und beispielsweise synthetische und/oder natürliche Öle, wie z.B. Triglyceride der Linolsäure, O/W-Emulgatoren, vorzugsweise Stearinsäure, neutralisiert mit TEA oder NaOH, und ggf. einen Coemulgator, mehrwertige Alkohole, vorzugsweise Butandiol oder Propandiol, Pigmente und / oder Perlglanzpigmente (Eisenoxide, Ultramarinblau, Chromhydroxidgrün), Füllstoffe, vorzugsweise Talkum oder Mica und gegebenenfalls auch Hilfsstoffe, wobei das Synthetische Wachs ein Fischer-Tropsch Wachs mit einer Kettenlänge von 23-64 Kohlenwasserstoff Atomen, einer Molmasse von > 300 g/mol und einer Viskosität von > 5 mm²/s bei 99°C ist.

Gegebenenfalls können zu der erfindungsgemäßen Formulierung auch Fasern wie z.b. Polyamid-Fasern oder Elastofasern, insbesondere Lycra ® zur optischen Verschönerung der Wimpern eingesetzt werden.

Die nachfolgend aufgelisteten Wachse (optionalen Wachse) können neben den synthetischen Esterwachsen, Bienenwachs und Synthetikwachs bevorzugt zusätzlich im Sinne der vorliegenden Erfindung verwendet werden:

| Klasse | Untergruppe | Beispiele |
|---|---|---|
| synthet. Wachse | Hartwachse | Montanesterwachse die keine synthetischen Esterwachse sind |
| natürliche Wachse | pflanzliche Wachse | Candelillawachs, Carnaubawachs, Japanwachs, Espartograswachs, Korkwachs, Guarumawachs, Reiskeimölwachs, Zuckerrohrwachs, Ouricurywachs, Montanwachs, Jojobawachs, Sonnenblumenwachs |
| | tierische Wachse | Schellackwachs, Walrat, Lanolin (Wollwachs), Bürzelfett |
| | Mineralwachse | Ceresin, Ozokerit (Erdwachs) |
| | petrochem. Wachse | Petrolatum, Paraffinwachse, Mikrowachse |

Es ist von Vorteil den Gehalt an synthetischen Esterwachsen, Bienenwachs und Synthetikwachs und optionalen Wachsen in erfindungsgemäßen Zubereitungen aus dem Bereich von 1 bis 20 Gew.-%, bevorzugt 5 bis 15 Gew.-% zu wählen.

Es kann auch vorteilhaft sein, daß die erfindungsgemäßen Mascarazubereitungen ein oder mehrere Pigmente enthalten, gewählt aus der Gruppe der Pigmente mit den C.I.-Bezeichnungen Pigment Red 57 : 1, Pigment Red 57 : 2. Pigment Red 172, Pigment Red 90 : 1, Pigment Yellow 100, Pigment Yellow 115, Pigment Red 174, Pigment Red 4, Pigment Blue 29, Pigment Violet 15, Pigment Violet 16, Pigment Red 29, Pigment Green 17, Pigment Green 18, Natural Red 4, Pigment White 6, Pigment White 14 und Pigment White 31. Pigment Blue 29, Pigment Violet 15, Pigment Violet 16, Pigment Green 18, Natural Red 4, Pigment White 6 und Pigment White 31..

Die Mascarazubereitungen enthalten die Pigmente bevorzugt in Mengen von 2-15 Gew.-%, insbesondere von 5,0-10,0 Gew.-%, jeweils bezogen auf die gesamte Zubereitung.

Gemäß einer bevorzugten Ausführungsform der Erfindung enthält die Haar-Mascara noch einen oder mehrere Weichmacher. Bevorzugte Weichmacher sind Polyethylenglykole, Polypropylenglykole, Polyglycerine sowie deren Mischungen. Besonders bevorzugt sind Polyethylenglykole mit mittleren Molmassen von 100 bis 4000, insbesondere 200 bis 1500. Polyethylenglykole mit mittleren Molmassen von 300 bis 600 sind erfindungsgemäß besonders bevorzugte Weichmacher. Aber auch Citronensäurealkylester und Acylcitronensäurealkylester wie Triethylcitrat, Tributylcitrat, Acetyltriethylcitrat und Acetyltributylcitrat können vorteilhaft Verwendung finden.

Zur Viskositätsoptimierung werden gern Verdicker wie beispielsweise Magnesium Aluminum Silicate (Veegum) Quaternium-18-hectorite (Bentone-38), substituierte und unsubstituierte Cellulosen und Stärkederivate eingesetzt.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 10 Gew.-%, vorzugsweise 0,05 bis 5 Gew.-%, jeweils bezogen auf das Gesamtgewicht der Zubereitung.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden. Feste Bestandteile werden hierzu in flüssigen Bestandteilen gelöst oder vor der Zugabe aufgeschmolzen.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

### Beispiel

| INCl | Menge % |
|---|---|
| Aqua | 50,05 |
| Cetearyl Behenate (Kesterwachs K62) | 6,0 |
| Synthetic Wax | 7,5 |
| Cera Alba (Binenwachs) | 2,0 |
| Stearic Acid | 6,0 |
| Cetyl Alcohol | 1,0 |
| Triethanolamine | 2,4 |
| Butylenglycol | 3,5 |
| Glycerin | 0,5 |
| Active ingredients | 1,55 |
| Cyclomethicone | 3,0 |
| Acrylates/Octylacrylamide Copolymer | 4,0 |
| Pigments (e.g. Iron Oxides) | 10,0 |
| Preservatives | 1,0 |
| Aluminum Starch Octenylsuccinate | 0,5 |
| Magnesium Aluminum Silicate | 1,0 |

## Patentansprüche

1. Mascarazubereitungen **dadurch gekennzeichnet, dass** sie eine Kombination aus mindestens einem Synthetikwachs, Bienenwachs und mindestens einem synthetischen Esterwachs, mindestens einen Filmbildner und mindestens einen Emulgator enthält, wobei das Synthetikwachs ein Fischer-Tropsch Wachs mit einer Kettenlänge von 23 - 64 Kohlenwasserstoffatomen, einer Molmasse von > 300g/mol und einer Viskosität von > 5 mm²/s bei 99°C ist.

2. Zubereitungen nach Anspruch 1, **dadurch gekennzeichnet, dass** das synthetische Esterwachs ein Veresterungsprodukt von langkettig gesättigten Fettalkoholen und Säuren pflanzlicher Herkunft ist und Ester mit Kohlenstoffkettenlängen zwischen 32 und 46, insbesondere 36 und 44, enthält.

3. Zubereitung nach mindestens einem der vorhergehenden Ansprüche, **dadurch gekennzeichnet, dass** das synthetische Esterwachs eine Molmasse von 500 bis 650 g/mol aufweist.

4. Zubereitungen nach mindestens einem der vorhergehenden Ansprüche, **dadurch gekennzeichnet, dass** der Gehalt an synthetischem Esterwachs 0,5 - 12,0 Gew.-%, bezogen auf das Gesamtgewicht der Zubereitungen , bevorzugt 2,0 - 10,0 Gew.-%, insbesondere von 4,0 - 8,0 Gew.-%, beträgt.

5. Zubereitung nach mindestens einem der vorhergehenden Ansprüche, **dadurch gekennzeichnet, dass** das Synthetikwachs zum überwiegenden Teil eine Kettenlänge zwischen 36 und 39 aufweist.

6. Zubereitungen nach mindestens einem der vorhergehenden Ansprüche, **dadurch gekennzeichnet, dass** der Gehalt an Synthetikwachses 0,5 - 12,0 Gew.-%, bezogen auf das Gesamtgewicht der Zubereitungen , bevorzugt 1,0 - 10,0 Gew.-%, insbesondere von 4,0 - 8,0 Gew.-%, beträgt.

7. Zubereitung nach mindestens einem der vorhergehenden Ansprüche, **dadurch gekennzeichnet, dass** das Bienenwachs weiß ist.

8. Zubereitungen nach mindestens einem der vorhergehenden Ansprüche, **dadurch gekennzeichnet, dass** der Gehalt an Bienenwachs 0,5 - 7,0 Gew.-%, bezogen auf das Gesamtgewicht der Zubereitungen , bevorzugt 1,5 - 6,0 Gew.-%, insbesondere von 2,5 - 5,0 Gew.-%, beträgt.

9. Zubereitungen nach mindestens einem der vorhergehenden Ansprüche, **dadurch gekennzeichnet, dass** der Gehalt an Wachsen 1,5 - 31 Gew.-% (Summe aller eingesetzten Wachse), bezogen auf das Gesamtgewicht der Zubereitungen , bevorzugt 4,5 - 26 Gew.-%, insbesondere von 10,5 - 21 Gew.-%, beträgt.

10. Verwendung von einer Kombination von mindestens einem Synthetikwachs, Bienenwachs und mindestens einem synthetischen Esterwachs in Mascarazubereitungen, wobei das Synthetikwachs ein Fischer-Tropsch Wachs mit einer Kettenlänge von 23 - 64 Kohlenwasserstoffatomen, einer Molmasse von > 300g/mol und einer Viskosität von > 5 mm²/s bei 99°C ist, zur Herstellung von bröckelarmen bzw. bröckelfreien wischfesten und Volumen gebenden Mascarazubereitungen.

## Claims

1. Mascara preparations **characterized in that** they comprise a combination of at least one synthetic wax, beeswax and at least one synthetic ester wax, at least one film former and at least one emulsifier, where the synthetic wax is a Fischer-Tropsch wax with a chain length of 23-64 hydrocarbon atoms, a molar mass of > 300 g/mol and a viscosity of > 5 mm²/s at 99°C.

2. Preparations according to Claim 1, **characterized in that** the synthetic ester wax is an esterification product of long-chain saturated fatty alcohols and acids of vegetable origin and comprises esters with carbon chain lengths between 32 and 46, in particular 36 and 44.

3. Preparation according to at least one of the preceding claims, **characterized in that** the synthetic ester wax has a molar mass of from 500 to 650 g/mol.

4. Preparations according to at least one of the preceding claims, **characterized in that** the content of synthetic ester wax is 0.5-12.0% by weight, based on the total weight of the preparations, preferably 2.0-10.0% by weight, in particular from 4.0-8.0% by weight.

5. Preparation according to at least one of the preceding claims, **characterized in that** the majority of the synthetic wax has a chain length between 36 and 39.

6. Preparations according to at least one of the preceding claims, **characterized in that** the content of synthetic wax is 0.5-12.0% by weight, based on the total weight of the preparations, preferably 1.0-10.0% by weight, in particular from 4.0-8.0% by weight.

7. Preparation according to at least one of the preceding claims, **characterized in that** the beeswax is white.

8. Preparations according to at least one of the preceding claims, **characterized in that** the content of beeswax is 0.5-7.0% by weight, based on the total weight of the preparations, preferably 1.5-6.0% by weight, in particular from 2.5-5.0% by weight.

9. Preparations according to at least one of the preceding claims, **characterized in that** the content of waxes is 1.5-31% by weight (sum of all waxes used), based on the total weight of the preparations, preferably 4.5-26% by weight, in particular from 10.5-21% by weight.

10. Use of a combination of at least one synthetic wax, beeswax and at least one synthetic ester wax in mascara preparations, where the synthetic wax is a Fischer-Tropsah wax with a chain length of 23-64 hydrocarbon atoms, a molar mass of > 300 g/mol and a viscosity of > 5 mm²/s at 99°C, for producing low-clumping or non-clumping wipe-resistant and volumizing mascara preparations.

## Revendications

1. Préparations pour mascara, **caractérisées en ce qu'**elles contiennent une combinaison d'au moins une cire synthétique, de la cire d'abeille et d'au moins une cire d'ester synthétique, d'au moins un agent filmogène et d'au moins un émulsifiant, où la cire synthétique est une cire de Fischer-Tropsch présentant une longueur de chaîne de 23-64 atomes de carbone, une masse molaire > 300 g/mole et une viscosité > 5 mm²/s à 99°C.

2. Préparations selon la revendication 1, **caractérisées en ce que** la cire d'ester synthétique est un produit d'estérification d'alcools gras saturés à longue chaîne et d'acides d'origine végétale et contient des esters avec des longueurs de chaîne carbonée entre 32 et 46, en particulier entre 36 et 44.

3. Préparation selon au moins l'une quelconque des revendications susmentionnées, **caractérisée en ce que** la cire d'ester synthétique présente une masse molaire de 500 à 650 g/mole.

4. Préparations selon au moins l'une quelconque des revendications susmentionnées, **caractérisées en ce que** la teneur en cire d'ester synthétique est de 0,5-12,0% en poids, par rapport au poids total des compositions, de préférence de 2,0-10,0% en poids, en particulier de 4,0-8,0% en poids.

5. Préparation selon au moins l'une quelconque des revendications susmentionnées, **caractérisée en ce que** la cire synthétique présente, pour la partie principale, une longueur de chaîne entre 36 et 39.

6. Préparations selon au moins l'une quelconque des revendications susmentionnées, **caractérisées en ce que** la teneur en cire synthétique est de 0,5-12,0% en poids, par rapport au poids total des compositions, de préférence de 1,0-10,0% en poids, en particulier de 4,0-8,0% en poids.

7. Préparation selon au moins l'une quelconque des revendications susmentionnées, **caractérisée en ce que** la cire d'abeille est blanche.

8. Préparations selon au moins l'une quelconque des revendications susmentionnées, **caractérisées en ce que** la teneur en cire d'abeille est de 0,5-7,0% en poids, par rapport au poids total des préparations, de préférence de 1,5-6,0% en poids, en particulier de 2,5-5,0% en poids.

9. Préparations selon au moins l'une quelconque des revendications susmentionnées, **caractérisées en ce que** la teneur en cires est de 1,5-31% en poids (somme de toutes les cires utilisées), par rapport au poids total des préparations, de préférence de 4,5-26% en poids, en particulier de 10,5-21% en poids.

10. Utilisation d'une combinaison d'au moins une cire synthétique, de cire d'abeille et d'au moins une cire d'ester synthétique dans des préparations pour mascara, où la cire synthétique est une cire de Fischer-Tropsch présentant une longueur de chaîne de 23-64 atomes de carbone, une masse molaire > 300 g/mole et une viscosité > mm²/s à 99°C, pour la préparation de préparations pour mascaras pauvres en grumeaux ou exemptes de grumeaux résistantes au frottement et conférant du volume.
